# EUROPEAN PATENT APPLICATION

(11) **EP 4 534 610 A1**
(43) Date of publication of application: **09.04.2025**
(21) Application number: 23201387.0
(22) Date of filing: 03.10.2023
(51) Int. Cl.: C09B 23/10, C09B 69/00, C09B 67/22

(54) **FLUOROPHORE COMPOUNDS AND PRODUCTION THEREOF**

(71) Applicant: Université Fribourg, 1700 Fribourg (CH)
(72) Inventor: Fromm, Katharina, 1700 Fribourg (CH); Vasylevskyi, Serhii, Lubbock, 79410 (US)
(74) Representative: P&TS SA (AG, Ltd.)

(57) **Abstract**

The invention concerns a novel fluorophore compound represented by any of the following General Formula P1, or General Formula P2, or General Formula P3 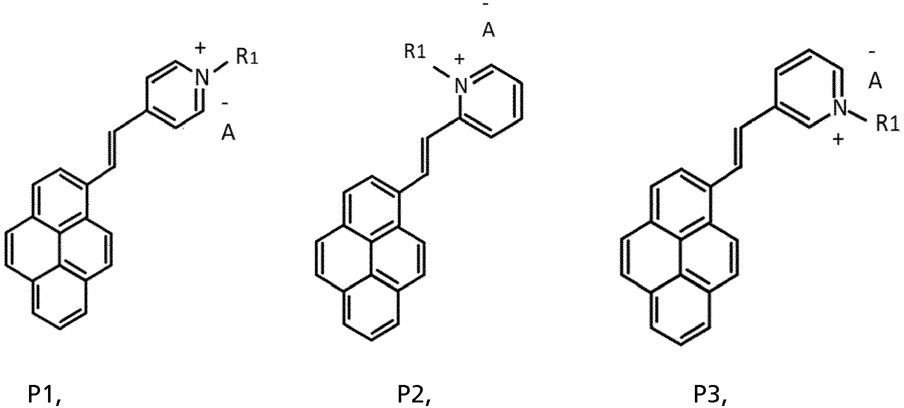 as well as a method for producing said compound and a staining composition comprising said compound.

## Description

### Technical domain

The present invention concerns family of novel organic fluorophores for staining cells and/or biological tissue, as well as the production of these fluorophores, as well as intermediate products.

### Related art

To gain a better understanding of the physiological processes and biological structures, fluorophore dyes are commonly used in imaging to stain cells or biological tissues. Different cell parts are usually stained with different dyes which absorb at unique wavelength, such as to avoid visual interference between the different staining agents. Good photostability under extended irradiation is a highly desirable feature for these dyes, as it permits for prolonged observation periods under laser irradiation scanning focal microscopy (LSCM) conditions. Imaging of living cells furthermore requires that the dyes have a low toxicity or are non-toxic to reduce the impact of the staining agent on the biological functioning of the cell.

Commonly used commercial dyes available today, such as Alexa Fluor 405, Alexa Fluor 488, Cascade blue, Atto 488 and Cyanine dyes, are complex compounds comprising a high number of functional groups. In addition, the functional groups of these dyes are usually sensitive and/or highly reactive. Moreover, the production of these dyes is complicated, requiring several protection steps of starting and/or intermediate products before and during the synthesis of the dyes. In particular the protection and deprotection steps are laborious and require close monitoring of the reaction conditions. It is therefore not only costly to produce these dyes, but also extremely difficult to scale their production. Moreover, a series of chromatographic purifications are usually required to obtain sufficiently pure dyes, further adding to the significant scaling challenges.

These conventional cell dyes also suffer from rapid bleaching and/or chemical degradation upon repeated irradiation, resulting in the loss of emission intensity within several minutes. Improved, more stable small dye molecules usually also contain complex functional groups, rendering the synthesis of these small dyes onerous and difficult.

To avoid photobleaching of organic dyes, the use of quantum dots based on nanoparticles with tuneable fluorescence has been explored. However, this approach necessitates expensive process steps, such as surface coating and functionalization, and is therefore economically not attractive. Yields are also typically low.

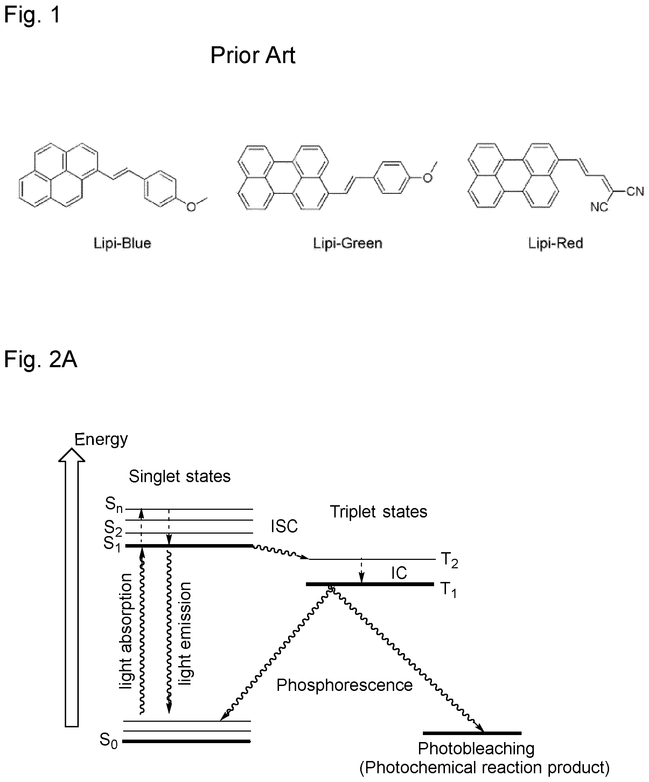

A series of less complex cell dyes as shown in Figure 1, which have distinct excitation wavelength is also commercially available.

In addition, Kodua Daniel et al, Journal of the American Chemical Society, 13 Apr 2022, vol 144, p 6343 to 6348 disclose the pyrenyl-based photochemically active compound pyridinepyrene (PyPe) which emits blue light when activated.

However, despite being more photo-stable than conventional dyes, the photostability of these less complex dyes is still sub-optimal.

The synthesis and production of non-photobleachable dyes for cell staining remains a significant challenge today. Preventive strategies of stabilizing the dyes and avoiding photobleaching by preventing their reaction with other molecules remain elusive today.

### Short disclosure of the invention

lt is an aim of the present invention to find a fluorescent reagent that overcomes one or more of the shortcomings and limitations of the state of the art.

It is another aim of the present invention to provide an organic fluorophore with improved photostability over known organic fluorophores. The fluorophore should sustain prolonged irradiation periods, allowing for longer observation of biological tissue or cells using LSMC.

It is another aim of this invention to provide a fluorophore dye having a simple structure compared to conventionally used dyes. The dye should preferably be capable of being produced at a high yield. The dye should be easy to purify.

Preferably, the production of the fluorophore dye should be readily scalable. The fluorophore dye should be scalable to kg production.

It is another aim of the invention to provide a photostable fluorophore dye which is suitable for staining living cells. The dye should preferably be more photostable that known dyes of similar chemical structure.

It is yet another aim of this invention to provide for an alternative fluorophore dye for staining biological tissue, cells, and/or biological molecules.

According to the invention, one or more of these aims are attained by the object of the attached claims, and especially a fluorophore compound represented by the following General Formula P1, or General Formula P2, or General Formula P3 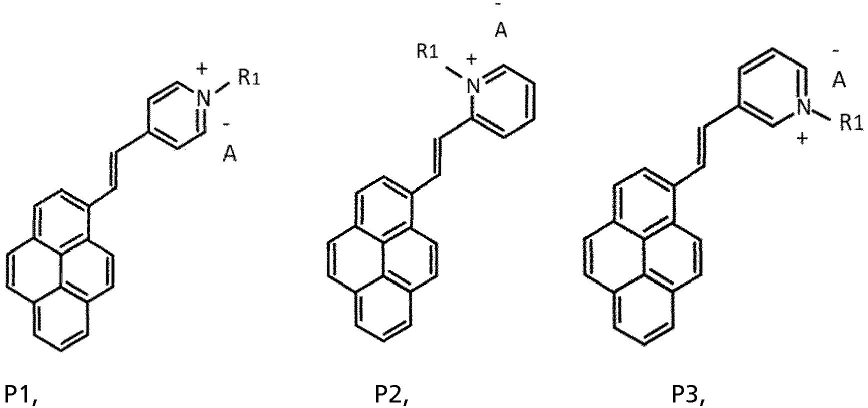
wherein R₁, denotes a methyl-group, or a linear or branched saturated or unsaturated alkyl group having from 2 to 10 carbon atoms, preferably 2, 3, 4, 5, or 6 carbon atoms, optionally substituted by 1, 2 or 3 groups independently selected from OR₂, NR₂R_{2'}, SR₂, an aryl group, an heteroaryl group, or a saturated or unsaturated cycloalkyl group having from 3 to 7 carbon atoms,
wherein R₂ and R_{2'} independently from each other denote H, a linear or branched saturated or unsaturated alkyl group having from 1 to 10 carbon atoms, preferably 1, 2, 3, 4, or 5 carbon atoms, optionally substituted by 1 to 3 groups independently selected from an aryl group, an heteroaryl group or a saturated or unsaturated cycloalkyl group having from 3 to 7 carbon atoms, and
wherein A⁻ denotes an anion selected from the group consisting of F, Cl⁻, Br⁻ , I⁻, tetrafluoroborate (BF₄⁻), hexafluorophosphate (PF₆⁻), triflate, tosylate, acetate, sulfate SO₄ ²⁻, OH⁻.

The compound of General Formulas P1 is the para-isomer, the compound of General Formulas P2 is the ortho-isomer and the compound of General Formulas P3 is the meta isomer of an N-functionalized pyridinepyrene (PyPe) derivative.

The compounds of General Formulas P1, P2 and P3 also comprise the individual stereoisomers, in which the stilbene-like linker motif can take an E-isomer configuration or a Z-isomer configuration. The General Formulas presented herein also comprise the mixtures of the respective stereoisomers.

The stilbene-like linker motif of the para-isomer has the following structure:

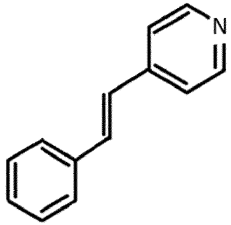

The N position in the stilbene-like linker motifs of the meta-isomer and ortho-isomer of the compound of this invention varies accordingly.

The *E*-isomer of a compound of this invention is also referred to as the trans configuration of the compound. The Z-isomer of a compound of this invention is also referred to as the cis configuration of the compound.

The E-isomer and Z-isomer for the para-isomer of a compound of this invention have the following structure:

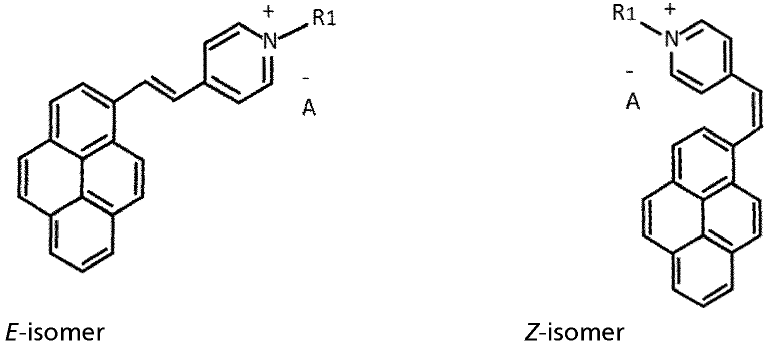

In analogy, the stilbene-like motif of the meta-isomer and the ortho-isomer of a compound of this invention can take the same the E-isomer and Z-isomer configuration as the above shown para-isomer.

The terms "PyPeA" and "compound of this invention" each refer to any or all compounds represented by the General Formulas P1, P2, or P3 as well all its stereoisomers, including its *E*-isomer and its *Z*-isomer presented above.

The term "PyPeBr" refers to a specific compound of this invention, in which the anion A⁻ is Br⁻.

In one aspect of this invention, the compound is provided in a solution comprising a mixture of its E- stereoisomer and its *Z*- stereoisomer. The mixture may comprise an excess of the *E*- stereoisomer. The mixture of said stereoisomers is preferably in a chemical equilibrium.

The chemical equilibrium, or the photo-stationary state, of a solution comprising the compound of this invention may be obtained by irradiating the compound at its excitation wavelength for a period of time to induce reversible flipping between the *E*- and *Z*- stereoisomers. The mixture therefore preferably comprises from 0 to 50% of *Z*- stereoisomers, depending on whether the mixture has reached its photo-stationary equilibrium.

In one aspect of this invention, the ratio between *E*-and *Z-*configurations obtained in the photo-stationary state of a dissolved compound is 50:50.

The photo-induced configuration switch from the *E*-stereoisomer to the *Z*- stereoisomers of known stilbene-derivatives is usually complete and irreversible. This irreversible conversion results in a gradual depletion of the *E*- stereoisomer in the mixture, thus preventing the establishment of a chemical equilibrium.

In contrast, the photo-induced configurational change between the *E*- and *Z*- stereoisomers of the compound of this invention is fully reversible. This reversibility enables the formation of a stable photo-stationary equilibrium, thereby preventing depletion of the *E-*stereoisomer. A compound of this invention is therefore more photostable than known stilbene derivatives.

The vinylene linker connecting the chromophore pyrenyl-panel with its pyridyl- R1 side group, contributes to the remarkable photostability of this compound. The vinylene-linker serves as a radiationless relaxation functional group, that leads to a very rapid configurational flip, preventing a reaction of the compound in its excited state with other molecules, for example oxygen or the solvent.

In addition, the positive charge of the bromide further accelerates the *E*/*Z* isomerisation of the compound of this invention and thereby contributes to the stability of the compound of this invention.

Steric hindrance due to the pyridyl group of the mono-substituted pyrenyl- group further contributes to the stabilization of the structure.

The N-functionalisation of the pyridyl-group with an R₁ group, for example an allyl-group, surprisingly proved to render the compound more stable at prolonged irradiation compared to 4-(2-(pyren-1-yl)vinylpyridine, hereinafter also referred to as "PyPe", which is an intermediate product in the synthesis of PyPeBr. This functionalisation therefore further reduced photobleaching of the fluorophore structure.

The compound of this invention is a novel compound.

The compound of this invention may be synthesized using PyPe as a starter material. PyPe may also be an intermediate product in the synthesis pathway. PyPeA, for example PyPeBr, is synthesized by performing an N-functionalisation of PyPe. This N-functionalisation is not trivial. During the introduction of the N-functional groups the product is highly reactive and susceptible to forming undesired end products.

In particular, it was found that PyPeA must be synthesized under dry conditions. To this end acetic anhydride is present in the reaction mixture.

The reaction conditions had to be diligently elaborated in research projects to obtain the desired N-functionalized end product and to obtain a satisfactory yield. In particular, it was found that the exclusion of oxygen during the reaction is essential for producing PyPeA.

The method for producing PyPeA is described in more detail below.

The functional group R₁ of the compound may be chosen to define its excitation wavelength and its emission wavelength. The compound of this invention therefore allows for the synthesis of fluorophores dyes having different optical properties, respectively emitting light in different colours.

In a preferred aspect, the compound of this invention is an allyl-pyridinium-4-yl bromide represented by General Formula P1.1

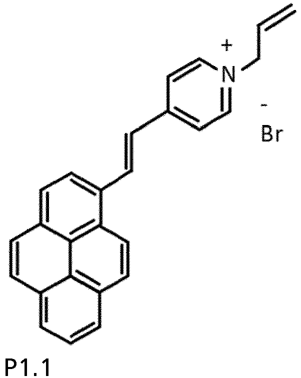

The compound of this invention has an excitation wavelength from 300 nm to 500 nm.

The compound of the General Formula P1.1 has a very broad emission range with emission peaks at 405, at 465nm,. and at 565nm.

While the overall appearance of the fluorophore dye of General Formula P1.1 is in the orange range of the visible light, optical filters may be used to detect emission peaks at different wavelength. The broad emission range of this compound therefore allows for selection of a specific emission wavelength for staining a biological entity. In other words, the colour of the dye may be modified by selecting the appropriate optical filter. The fluorophore dye of General Formula P1.1 is therefore extremely versatile and can readily be combined with other fluorescent dyes.

The compound of this invention may be used as a dye for staining biological tissue, cells, and/or biological molecules. Due to its low toxicity and limited interference with biological processes, it is particularly suited for staining living cells.

For staining of specific target molecules or structures the compound of this invention may be conjugated to an antibody, a recombinant antibody, or to another molecular targeting agent. The targeting agent may for example be a polypeptide targeting agent, or a small molecule targeting agent.

The compound of this invention may of example be conjugated with biotin, or with streptavidin, or with avidin.

Examples for an embodiment of such a conjugate is shown in the following as General Formula P1.2 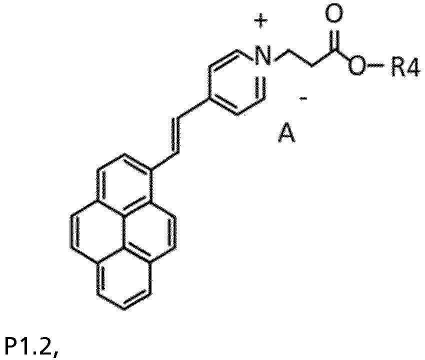 wherein R4 denotes biotin, streptavidin, or avidin.

Based on their specific binding properties of streptavidin and avidin to biotin, these biotin, streptavidin, and avidin fluorophore conjugates are particularly useful as research tools, for example to stain biological material linked to the complementary biotin, streptavidin or avidin molecules.

The compound of this invention may be dissolved in a polar protic and aprotic solvent, such as water, acetone, acetonitrile (CH₃CN), isopropanol, and methanol (MeOH), ethanol (EtOH), acetic acid, chloroform (CHCl₃), diethyl ether, ethyl acetate, methylene chloride, tetrahydrofuran (THF), dimethylformamide (DMF), dimelthylsulfoxide (DMSO), The compound of this invention may also be dissolved in a nonpolar solvent such as an alkanes, for example a pentane, a hexane, for example c-hexane (c-Hex) or a heptane, or an aromatic solvent, such as toluene (TOL), benzene, mesitylene (Mes), chlorobenzenes, , or pyridine. The compound may be dissolved in a mixture of different solvents.

The compound of this invention may be provided in a liquid composition as a staining reagent. The compound of this invention of a composition comprising the compound may for example used for *in vitro* or *ex vivo* staining of cells, biological tissue, or biomolecules.

The concentration of the compound in a staining reagent, as well as the amount used for staining may be appropriately changed in accordance with the biological entity or molecule to be stained, as well as in accordance with the desired staining intensity.

The compound of this invention may for example be added to a cell to a final concentration of 0.01 to 100 µM.

The compound of this invention may be produced by providing 4-(2-(pyren-1-yl)vinyl)pyridine, also referred to as PyPe, having the General Formula 10

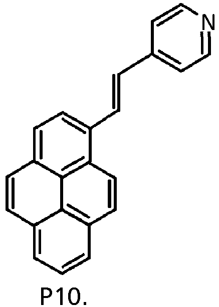

For the synthesis of PyPeBr PyPe is preferably provided in dried CH₃CN.

The dissolved PyPe is the mixed with preferably an excess amount of a product of the General Formula P11,

Br - R1 P11.

In one embodiment product P11 is allyl-bromide.

In a subsequent step dry acetic anhydride is provided to the reaction mixture, which it then aged under dry conditions above ambient temperature for a period of time, for example at a temperature ranging from 50°C to 130°C, or from 50°C to 80°C.

The terms "dry", "dried", or "dry condition" refer to the absence of water from a product, solvent, reagent, or a process.

To precipitate the product of the General Formula P1, the temperature of the reaction mixture was lowered to ambient temperature of about 25°C. 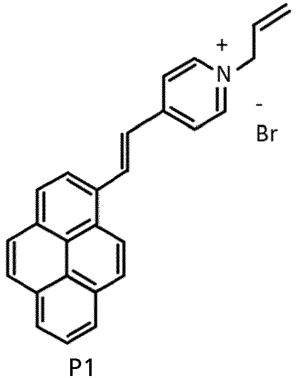 wherein R₁ is as above defined.

Importantly, the reaction needs to be performed under dry conditions. For this purpose, it is advisable to flush the reaction vessels several times with Argon (Ar or N₂) before adding the products and solvents. Dried CH₃CN is preferably added to the reaction vessels through a cannula.

It is furthermore advisable to heat the reaction mixture under reflux conditions while stirring vigorously.

Optionally, the completeness of the reaction can be tested by Thin-Layer Chromatography (TLC).

To prevent photobleaching during the synthesis of the PyPeBr product and to optimise its yield, the reaction should be performed in the dark, i.e. in the absence of light, in particular light in the visible range.

For the introduction of a product P11, in which R1 is not methyl-group, the synthesis should be carried out in the dark. In particular, if R1 is an allyl-group or similarly reactive group, it is essential that the reaction is carried in the dark. However, a methyl-group may be introduced in the presence of light.

The introduction of a product P11 should be performed slowly, since rapid addition of the group may lead to side product formation and polymerisation of the compound PyPe.

For purification, the precipitated product may be filtered and, optionally, washed. Preferably the precipitated product in washed under dry conditions. It may be washed under reduced pressure conditions. The precipitated compound may be washed with an aliphatic solvent, for example with n-pentane.

The yield of the dried final PyPeBr product synthesised according to this method from the PyPe intermediate product is high. It may be at least 90%, or at least 95% or at least 98%.

Optionally, the intermediate product PyPe may be synthesised by performing the steps described in the following.

Typically, molecules of a similar type to PyPe are synthesiszed through a Horner-Wadsworth Reaction. It would therefore be obvious to synthesise PyPe through this reaction. This synthesis is however not very cost-effective and is also difficult to carry out.

To overcome these drawbacks an alternative, improved method for the synthesis of PyPe was developed. Importantly, the drawbacks mentioned above were overcome by synthesizing PyPe in the presence of acetic anhydride in the reaction mixture. Acetic anhydride scavenges the water which is formed during the PyPe synthesis.

A product of General Formula P20 is mixed with a product of General Formula P21.1, P21.2, or P21.3 under dry reaction conditions

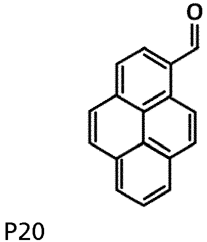

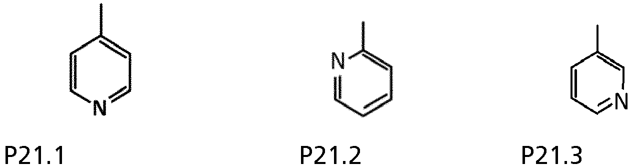

Dry acetic anhydride is added to the mixture, which is then aged for a period of time at a temperature. A suitable temperature for aging may for example be 80°C to 120°C. The mixture may be aged for 9 to 12 hours, preferably for 10 to 12 hours.

The aged mixture is subsequently cooled to ambient temperature, for example to 25 °C ± 3 °C, to precipitate the intermediate product represented by the General Formula 10.1, respectively by the General Formula 10.2, or the General Formula 10.3.

Compared to conventional complex cell dyes, the simpler pyrenyl-based structure of the compound of this invention provides the advantage that it requires less protection and deprotection steps for its synthesis and is therefore easier and less costly to produce.

In addition, the purification of the compound of this invention is extremely simple. Since its pyrenyl-structure is very poor in polar functions, the compound of this invention is easy to precipitate. The precipitate can be purified by simple filtration, a very straightforward process which is suitable for scaling.

The compound of this invention is remarkably photostable and resistant to photobleaching due to its reversible *E*/*Z*-isomerisation, which is further improved by the N-functionalisation of the pyridyl group.

As a further advantage, the compound of this invention is a salt and as such solvable in aqueous solutions. In addition, the compound of this invention can be dissolved in organic solvents. It is therefore extremely versatile and can be used for a broad range of different staining applications.

### Short description of the drawings

Exemplar embodiments of the invention are disclosed in the description and illustrated by the drawings in which:
**Figure 1** depicts cell dyes of relatively simple structures known in the art;
**Figure 2A** is a Jablonski diagram depicting the mechanism of photobleaching of typical dyes;
**Figure 2B** is shows light-induced (hv) configuration change for *E, Z-*stilbene, involving equilibrium of isomers and the irreversible photoproducts cyclobutyl-derivative (C) and dihydrophenantrene (D);
**Figure 3** shows the general formulas of the stilbene-like motif compounds tested in this study: PyPe, an intermediate product in the synthesis of a compound of this invention, PyPeBr, a final product of this invention, and PyPh, a related compound lacking the nitrogen (N) heteroatom;
**Figure 4A** shows a photoluminescence emission spectra collected of PyPe in different solvents upon excitation at 370 nm;
**Figure 4B** shows photoluminescence emission spectra collected of PyPeBr in different solvents upon excitation at 440 nm when dissolved in CH₃CN, CHCl₃, DCM, DMF, MeOH, Tol, respectively at 370nm when dissolved in c-Hex an in THF;
**Figure 4C** shows photoluminescence emission spectra collected of PyPh in different solvents upon excitation at 370 nm;
**Figure 5A** depicts the solid-state crystal structure of PyPeBr as determined by X-ray analysis;
**Figure 5B** shows light-induced *E*/*Z*-isomerisation of PyPeBr, whereby the allyl-group of PyPeBr also rearranges its orientation from *E*-allyl to Z-allyl to adopt a lower energy configuration after irradiation with lightwaves at 400 nm;
**Figure 6A** shows UV-Visible spectra of 5µM of PyPeBr in MeOH collected after continuous irradiation with 400 nm light;
**Figure 6B** shows UV-Visible spectra of 5µM of PyPeBr in DMF collected after continuous irradiation with 400 nm light;
**Figure 6C** shows UV-Visible spectra of 5µM of PyPeBr in ultra-pure water (MilliQ purification) collected after continuous irradiation with 400 nm light;
**Figure 6D** shows UV-Visible spectra of 5µM of PyPe in MeOH collected after continuous irradiation with 400 nm light;
**Figure 6E** shows UV-Visible spectra of 5µM of PyPe in DCM collected after continuous irradiation with 400 nm light;
**Figure 6F** shows UV-Visible spectra of 5µM of PyPh in MeCN collected after continuous irradiation with 400 nm light;
**Figure 6G** shows UV-Visible spectra of 5µM of PyPh in MeOH collected after continuous irradiation with 400 nm light;
**Figure 6H** shows UV-Visible spectra of a 5µM solution of Cascade Blue in in ultra-pure water (MilliQ purification) collected after continuous irradiation with 400 nm light;
Figure 7A shows a ¹H-NMR spectra of 2mg of PyPe in *d₄*-MeOD obtained after irradiation of solution for 0, 5, 15 and 30 min with a Xenon-arc lamp (300 Watt, Filter 400 nm, FWHM 25 nm), with the observed *E*/*Z*-isomerization of PyPe shown on the right;
**Figure 7B** shows a ¹H-NMR of 2 mg of PyPeBr in d4-MeOH under irradiation of 0, 1, 5, 15, and 30 min with excitation light at 400 nm (Xenon-arc lamp; 300 Watt, Filter 400 nm, FWHM 25 nm), with the light-induced E/Z isomerization of the allyl-group of PyPeBr shown above the spectra;
**Figure 7C** shows ¹H-NMR of 2mg of PyPh in CDCl₃ after irradiation for 0, 15 and 30 min with excitation light at 400 nm (Xenon-arc lamp 300 Watt, Filter 400 nm, FWHM 25 nm) with the observed formation of new compound that might correspond to a self-photobleached product shown in the inset;
**Figure 8A** shows PyPe, PyPh and PyPeBr fluorescence emission spectrum in situ excited at 405 nm, showing a peak at 465 nm (PyPe), 450 nm (PyPh) and 565 nm (PyPeBr) *in situ;*
**Figure 8B** shows a low magnification fluorescence image of PyPeBr after loading in the HEK cells and excitation *in situ* at 405 nm;
**Figure 8C** shows a high magnification fluorescence image of *OVA-B16* cells stained with PyPe showing cytosolic staining and subcellular partitioning.

### Examples of embodiments of the present invention

Rapid photobleaching is an enormous limitation for long-term exposure cell imaging.

Photobleaching occurs when light is first absorbed by a molecule, thereby promoting it into an energetically excited state, and subsequently dissipated into emission of light and movement, respectively vibrations, in the molecule. If the vibrations are too strong, the molecular bonds of the molecule break and the dye is destroyed. This bleaching process commonly occurs within a few minutes of irradiation.

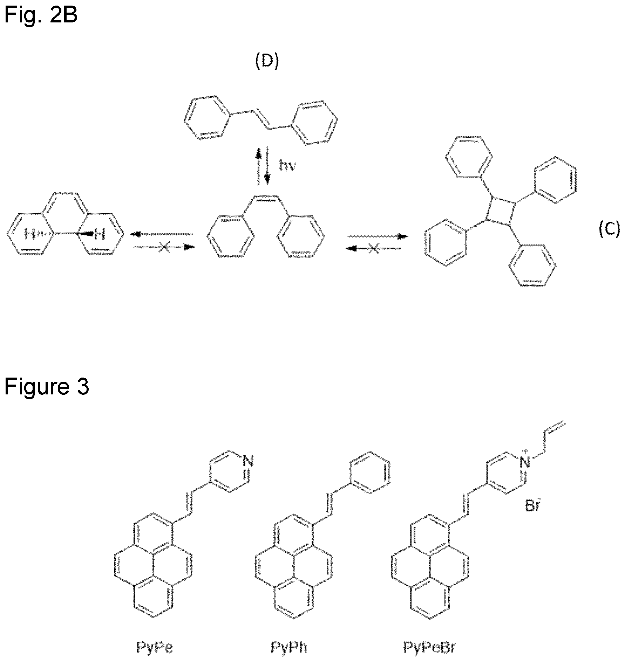

The photobleaching process of fluorophores is schematically illustrated in Figure 2, showing a Jablonski diagram. Irradiation of the molecule transfers it from its ground state S₀ into the excited single state (S₁, S₂, Sₙ), upon which intersystem crossing (ISC) to a forbidden excited triplet state (T₁, T₂, Tₙ) is likely to occur. In this forbidden triplet state the chromophore is extremely reactive and highly susceptible to react with other dye molecules or with molecules of the environment, such solvent molecules or oxygen molecules, resulting in a photobleached dye.

(*E*)-Stilbene itself is well known as a precursor to derivatives used as fluorophore dyes. Extended irradiation of stilbene-derivatives usually results in loss of the conjugation through vinylene-linker as a result of dimerization of the stilbene-derivative through its vinylene- bonds in its excited state.

The light-induced configuration change from *E*-stilbene to *Z*-stilbene, involving equilibrium of isomers, results in the formation of the irreversible photoproducts cyclobutyl-derivative and dihydrophenanthrene as shown in Figure 2B. For fluorophore dyes containing the stilbene-like motif, analogous irreversible reactions cause a depletion of the fluorophore compound and thereby bleaching of the dye.

To find a stable fluorophore dye which is resistant to photobleaching, a novel pyrenyl-based dye with the vinylene- functional group, (E)-1-allyl-4-(2-(pyren-1-yl)vinyl)pyridinium bromide (PyPeBr), was synthesised (Figure 3A). The photostability of this novel PyPeBr compound was compared to other stilbene-derivatives with similar structure, specifically (E)-4-(2-(pyren-1-yl)vinyl)pyridine (PyPe) (Figure 3B), which is an intermediate product in the synthesis of PyPeBr, and to (E)-1-styrylpyrene (PyPh) (Figure 3C).

The vinylene-linker of these compounds enables the configuration motion swap when the compounds are in the excited states causing the dye from the triplet-excited state (Tₙ*), in which they are highly reactive, to the ground state. The shorter the compounds remain in their triplet-excited state (Tₙ*), the less likely they are to react chemically with surrounding molecules.

Moreover, steric hindrance of mono-substituted pyrenyl-chromophore functional group further stabilize the dye thereby allowing high degree of freedom at Tn*. A key factor for the high absorption and photo-stability of the pyrenyl-chromophore is the high intersystem crossing ("ISC") efficiency of PyPe, respectively PyPeBr. Since the configurational change due to ISC occurs in a shorter time frame than chemical reactions leading to the formation of photobleaching products, PyPe and PyPeBr remain stable upon irradiation. The dyes are therefore less susceptible to depletion, or not depleted when exposed to light and photobleaching is significantly reduced or prevented.

*E*- and *Z*- configurations of PyPe both have a similar absorption spectrum when in the photostationary state, i.e. in their chemical equilibrium, with excitation wavelength ranging from 250nm to 420nm. The *E-* and *Z-* configurations of PyPe absorb a similar quantity of photons within this broad range. PyPe is particularly suited for long-term imaging with minimal or no photobleaching due to its radiationless pathway of energy release when transitioning from the excited Tₙ*/S* states to its S⁰ ground state.

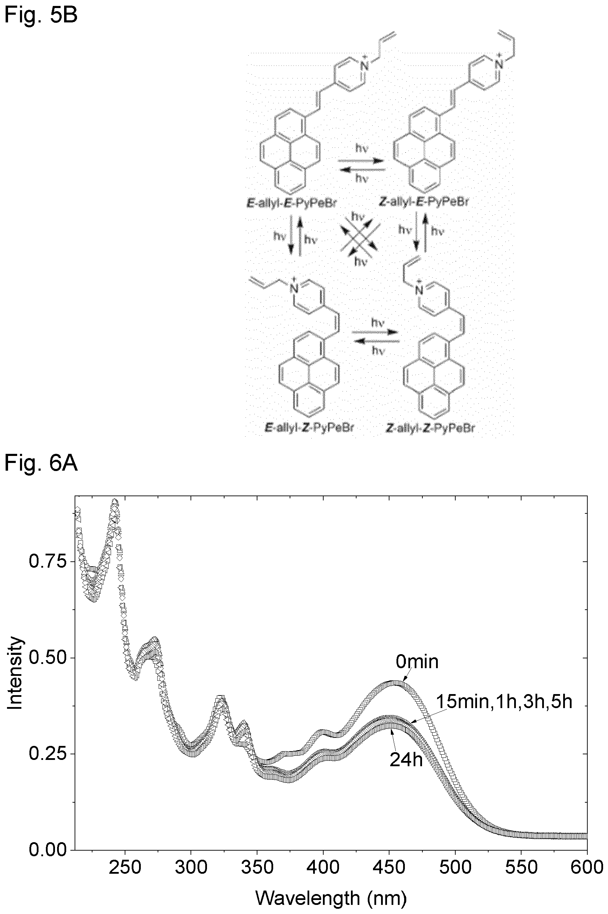

The photo-physical properties of these dye molecules with stilbene-like motifs were investigated in different solvents in order to determine their maximum absorption and emission wavelengths (Figures 6A to 6C). The compounds were dissolved in Acetonitrile (CH₃CN), chloroform (CHCl₃), c-hexane (c-HEX), dichloromethane (DCM), dimethylformamide (DMF), methanol (MeOH), tetrahydrofuran (THF), and toluene (Tol).

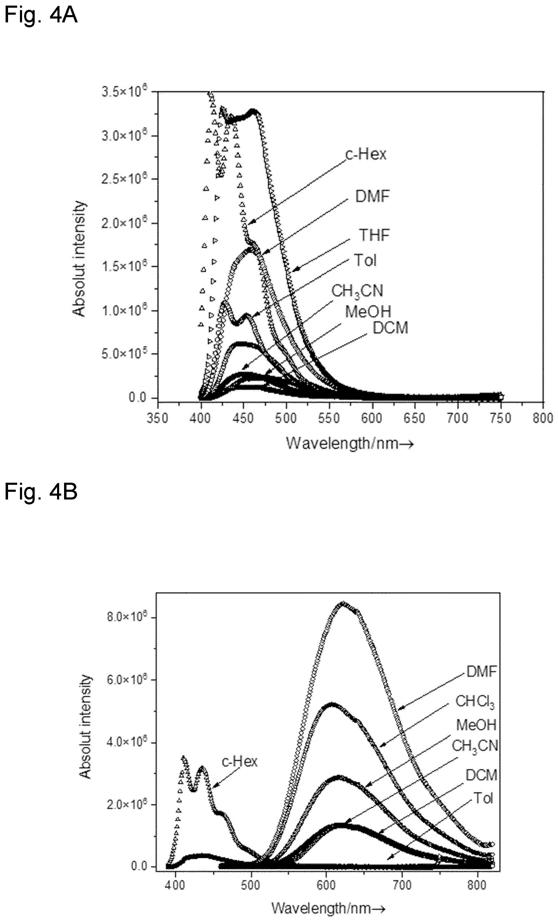

Figure 4A shows photoluminescence spectra of dissolved PyPe upon excitation at 370nm. The highest emission intensity was detected when THF and c-Hex were used as solvents, with lesser intensity recorded for DMF and Tol. PyPe emits light in the blue range, with emission peaks between 380nm and 550nm. Absolute intensities of up to 3.5 x 10⁶ were recorded for PyPe in c-Hex.

The photoluminescence spectra of PyPeBr upon excitation at 440nm when dissolved in CH₃CN, CHCl₃, DCM, DMF, MeOH, or Tol, and upon excitation at 370 nm when dissolved in c-Hex or THF is shown in Figure 4B. PyPeBr has a relatively broad emission spectrum, which depends on the solvent used. The emission spectrum of PyPeBr can be as broad to cover most of the visible range. This broad emission range offers the possibility to select a desired wavelength for imaging by applying appropriate filters.

Since the emission spectra of PyPeBr are relatively broad, it is also possible to use optical filters, in order to select a desired wavelength for imaging. PyPeBr can therefore be readily combined with other dyes for staining cells or tissues, wherein the width and variability of the PyPeBr emission spectra allow for combining dyes without or with reduced interference of emission spectra.

When dissolved in c-Hex or THF, PyPeBr emits light in the blue range, from 400nm to 500nm when excited at 370.

When dissolved in an of the other tested solvents and excited at 440nm, PyPeBr emitted light in the orange/red range, from 550nm to 750 nm.

The solvent-dependent variability in emission spectra of PyPeBr provides for a broad range of suitable applications, as the desired or optimal emission spectrum of the dye can be chosen for a specific application by simply choosing the solvent. This can be further optimised by selecting both the solvent and a filter to achieve the desired wavelength and intensity for a read-out.

It was furthermore be observed that the overall emission intensity of PyPeBr solutions was generally higher when compared to the PyPe solutions, with the highest intensity of over 8.5 x 10⁶ obtained for PyPeBr dissolved in DMF.

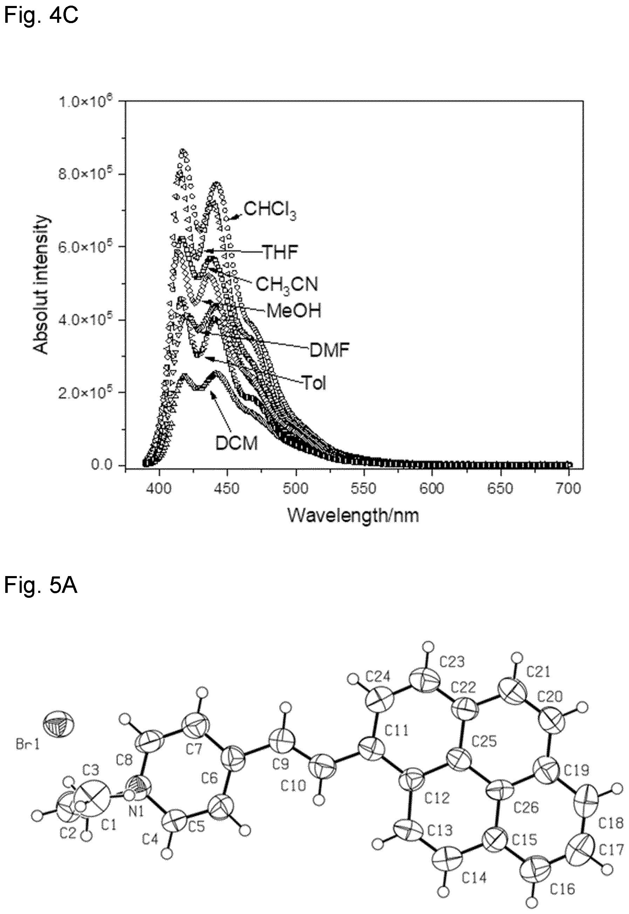

Figure 4C shows photoluminescence spectra of dissolved PyPh upon excitation at 370nm. PyPh emits light in the blue range, ranging from 400nm to 500nm. However, compared to PyPe and in particular to PyPeBr the light emission intensity is significantly reduced, with highest emission intensity of PyPh being about a factor 1000 less than optimal emission intensities of PyPeBr. The results demonstrate that PyPh is considerably less photostable than PyPe. The loss in stability is due to the self-photobleaching reactions, which are induced by irradiation.

Evidence of the efficient photostationary state of PyPe, was provided by exposing a coordination metal complex of *E*-PyPe with Zn²⁺, [Zn(*E-*PyPe)₂Cl₂]·2DCM, comprising two PyPe. X-Ray analysis confirmed that prior to irradiation both PyPe in the complex were primarily in *E*-configuration when coordinated to the Zn²⁺ metal centre.

Following irradiation with 400 nm light of 300W for 24 h using a Xenon arc lamp (FWHN = 25 nm), the X-Ray structure of a complex isolated from the irradiated solution revealed that one of the PyPe in the complex had changed its configuration to *Z-* while the other PyPe remained in its *E-*configuration, resulting in a [Zn(*E*-PyPe)(*Z*-PyPe)Cl₂] complex.

This experiment provided evidence that the light-induced *E*/*Z-*configurational change in PyPe reaches a photostationary equilibrium of *E*/*Z*-PyPe. The result also indicates that a 50%:50% ee ratibetween *E-* and *Z-*configurations is obtained in the photostationary state, further supporting previous results obtained from NMR and UV-Vis spectra, as mentioned below.

The photo-stable PyPe compound was further modified by introducing a positive charge to its pyridyl-group.

This was achieved by reacting allyl bromide at 40 °C in Acetonitril (MeCN) as described below to produce PyPeBr. Interestingly, it was observed that the *E*/*Z*-isomerization in PyPeBr takes place substantially faster than in PyPe at similar irradiation conditions as observed NMR and UV-visible experiments.

The increase in ISC efficiency for PyPeBr can be explained by the positive charge delocalization at the excited states (S*, Tₙ*). In addition, the presence of the heavy Br⁻ anion increases the spin-orbital coupling and leads to higher efficiency of ISC compared to PyPe.

Surprisingly, the N-alkylation of pyridyl- group with an ally-group does not result in faster photobleaching of PyPeBr compared to the PyPe intermediate product. Instead, it was observed that it renders the dye more stable at prolonged irradiation with 400 nm light.

Different from its PyPe intermediate product, PyPeBr is not only soluble in organic solvents but, due to its polar nature, it is also highly soluble in water.

Low water solubility is a major disadvantage compromising the use of many fluorescent dyes for staining of biological tissues or cells. The good water-solubility of PyPeBr is therefore an important advantage for this application.

The solid-state crystal structure of PyPeBr as determined by X-ray experiments is shown in Figure 5A. The allyl-group rearranged its orientation from *E*-allyl- to *Z*-allyl, as is shown in Figure 5B, upon irradiation with 400 nm light of 300 W using a Xenon arc lamp. Figure 5B depicts photoproducts that were detected in PyPeBr solutions by ¹H-NMR.

To examine the stability of the absorbance of PyPeBr and PyPe, 5µM solution (2.5 mL) of the respective compounds were prepared using different solvents. The compounds were then irradiated with 400nm using a Xenon-arc lamp at 300 Watt (25 nm FWHM). Following predefined irradiation periods, indicated in Figures 6A to 6H, UV-visible spectra were collected to monitor the stability of PyPeBr and PyPe in different solvents.

Figure 6A shows UV-Visible spectra of a 5µM solutions of PyPeBr in MeOH following 0min, 15min, 1h, 3h, 5h and 24h irradiation with 400 nm light. PyPeBr proved to be remarkable photostable with only a minor decrease in absorbance after 15 minutes of irradiation but no further significant increase over a 24h irradiation period.

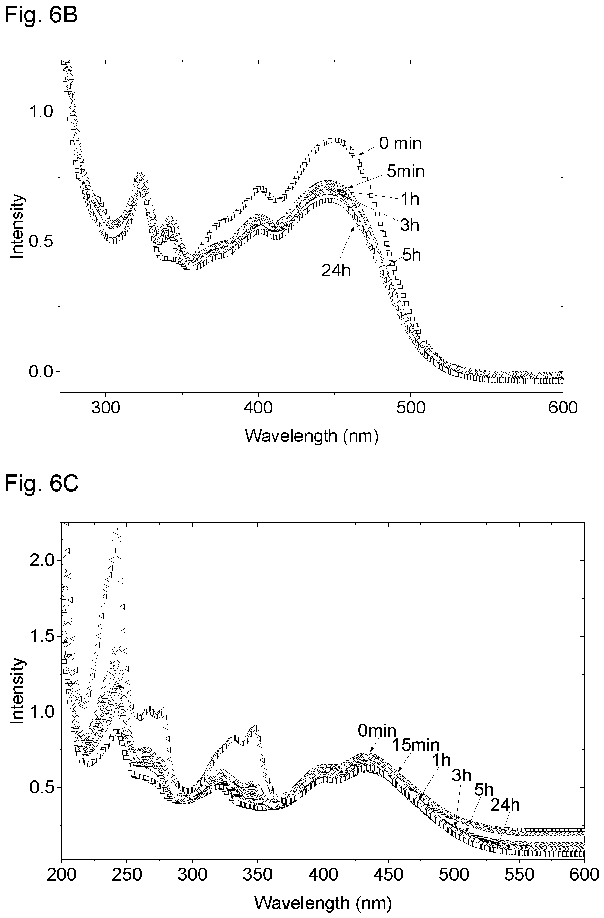

Similar results were obtained for PyPeBr in a DMF solution as shown in Figure 6B. Similar results were also obtained when PyPeBr was dissolved in MeCN (data not shown).

The capacity of PyPeBr to absorb light ranging from 370 nm to 500 nm remained substantially unchanged over a 24h irradiation period when PyPeBr was dissolved in ultra-pure water (MilliQ purification) as shown in Figure 6C.

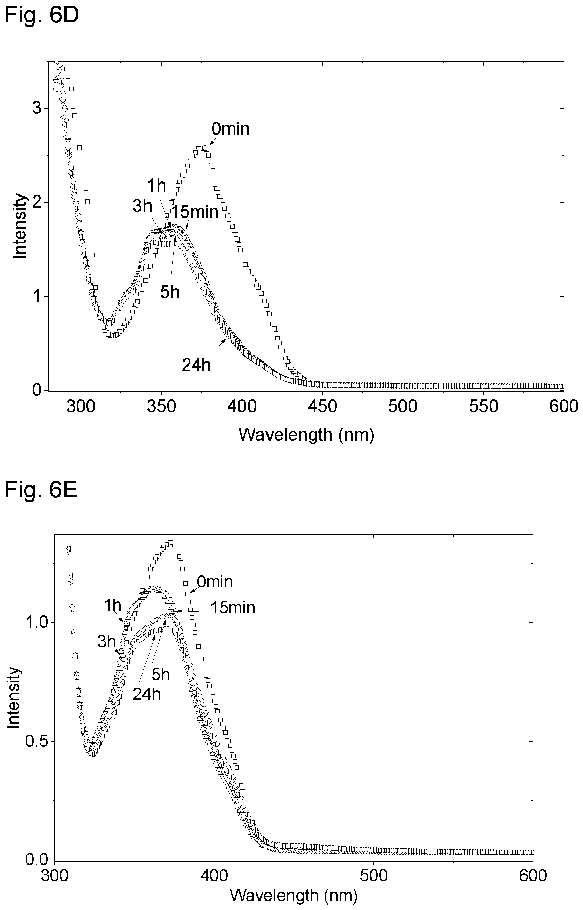

Figures 6D and 6E show absorption spectra collected at different times over a 24 hours irradiation period of PyPe dissolved in MeOH (Figure 6D) and in DCM (Figure 6E) respectively. While this compound also showed a good photostability, an early bleaching effect could be detected within the first 15 minutes of irradiation with 400 nm light.

Taken together these results clearly indicate an improved photostability of PyPeBr compared to PyPe, which already achieves good photostability.

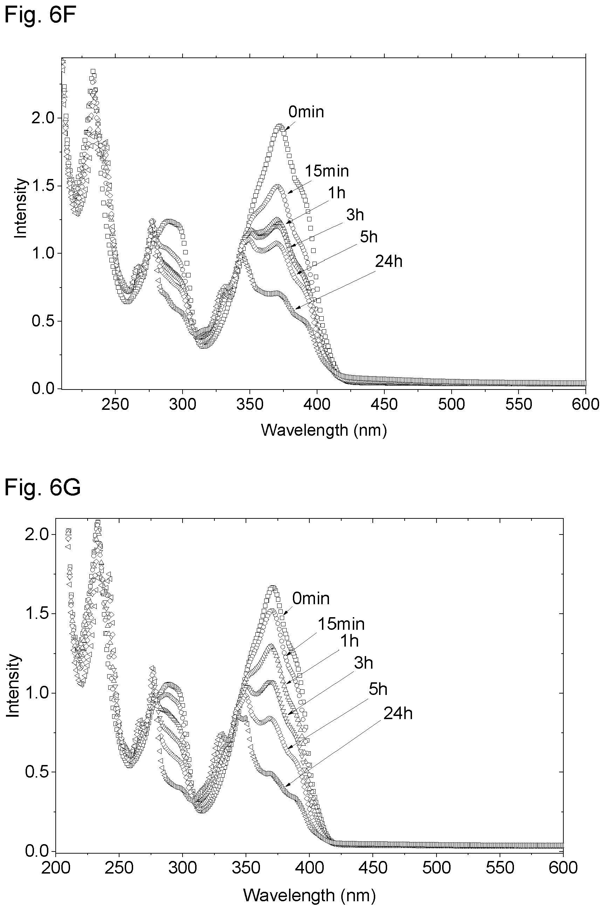

PyPh, which also comprises a stilbene-like motif, was also tested using similar experimental setups (Figures 6F and 6G).

Figure 6F shows UV-Visible spectra collected after continuous irradiation of 5µM of PyPh dissolved in MeCN for 24h with 400 nm light for the indicated times. Figure 6G shows the equivalent spectra of PyPh dissolved in MeOH. Both experiments clearly demonstrate that the absorption capacity, and therefore the stability of PyPh gradually decreases over a 24h irradiation period, with a significant drop detected as early as after 15 mins of irradiation. Comparable results were obtained for UV-Visible spectra collected from PyPh dissolved in DCM (data not shown).

Interestingly, PyPh demonstrated considerably lower photostability than PyPe and PyPeBr. This reduced photostability is due to the self-photobleaching.

However, the stilbene-like motif still provides a relatively good stability of PyPH when compared to other commercially available fluorescent dyes, such as Cascade Blue.

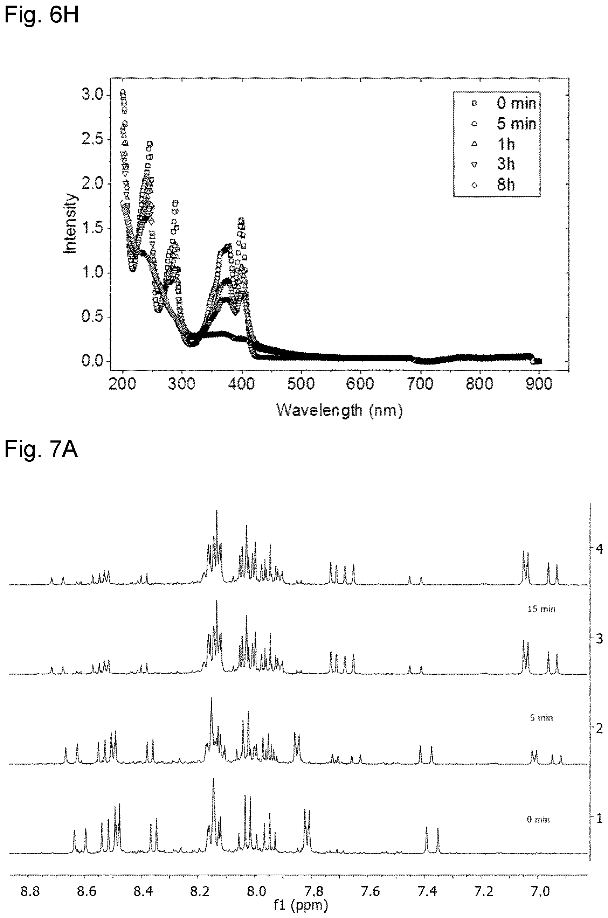

Cascade Blue, was submitted to the same experimental conditions. A 5µM solution of 2.5ml Cascade Blue dissolved in ultra-pure water (Milli Q purification) was irradiated with 400 nm light for 24h. Complete photobleaching of this compound was already observed after 8h of irradiation, as shown in Figure 6H.

It could be demonstrated that both PyPeBr as well as PyPe have very good photostability, which is considerably higher than the already good stability of PyPh. PyPeBr shows an even better resistance to photobleaching, respectively improved photostability, than PyPe.

The configurational change from the *E-* to *Z-* form of the stilbene-like motif, which contributes to the photostability of the PyPe and PyPeBr compounds, could be observed in ¹H-NMR profiles.

Figure 7A shows ¹H-NMR spectra of 2mg of PyPe dissolved in tetradeuteromethanol (*d*₄-MeOD) collected after irradiation at 400 nm (Xenon-arc lamp, 300W, FWHM 25nm) for 0, 5, 15 and 30 min. The chemical shifts between 7.9ppm and 7.4ppm indicate the *E*/*Z* isomerization of PyPe. The configurational change of the *E*-isomer of PyPeBr to its *Z*-isomer is indicated by the light-induced reaction shown next to the NMR spectra in Figure 7A.

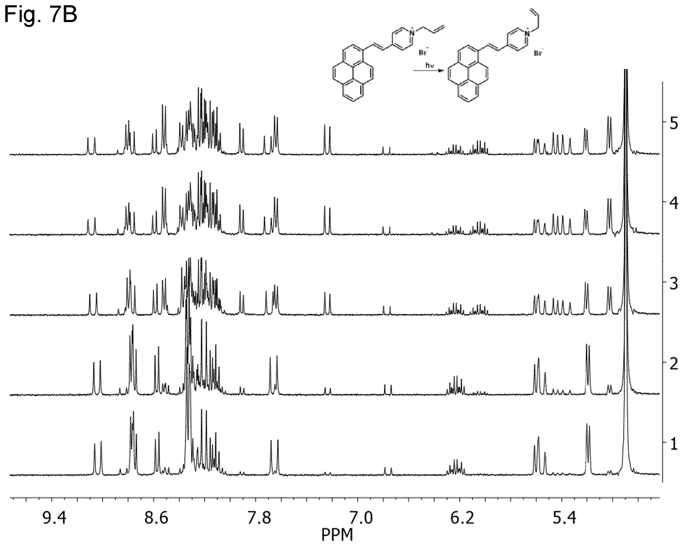

The same shifts can be observed in the ¹H-NMR spectra of irradiated PyPeBr, as shown in Figure 7B.

Interestingly, it was found that the allyl-group of PyPeBr also rearranges its orientation as a result of irradiation thereby adopting an energetically lower configuration. This second configurational change may contribute to the PyPeBr's improved photostability when compared to PyPe.

The additional configurational changes of the PyPeBr allyl-group were observed in NMR spectra of the irradiated PyPeBr samples in tetradeuteromethanol (d4-MeOD). NMR spectra taken after 0 mins, 1min, 5 mins, 15 min, and 30 mins of irradiation with 400nm are shown. The new chemical shifts can be seen at the range of 6.5 - 5.0 ppm for allyl-group *E*/*Z-*isomerization. The configurational change of the allyl-group of PyPeBr is indicated by the light-induced reaction shown above the NMR spectra in Figure 7B.

This configurational change of the PyPeBr allyl-group was also observed in the crystal structure of PyPeBr obtained from X-ray analyses of an isolated taken after irradiation (data not shown).

In order to better understand the exceptional photostability of PyPe, the compound was redesigned to exclude an influence of the heteroatom N, by introducing a conjugation with the phenyl-ring, to obtain compound PyPh shown in Figure 3C.

Interestingly, PyPh demonstrated considerably lower photostability due to the self-photobleaching, as evidenced in UV-visible analysis mentioned above and shown in Figures 6F and 6G. These results were further supported by NMR analyses.

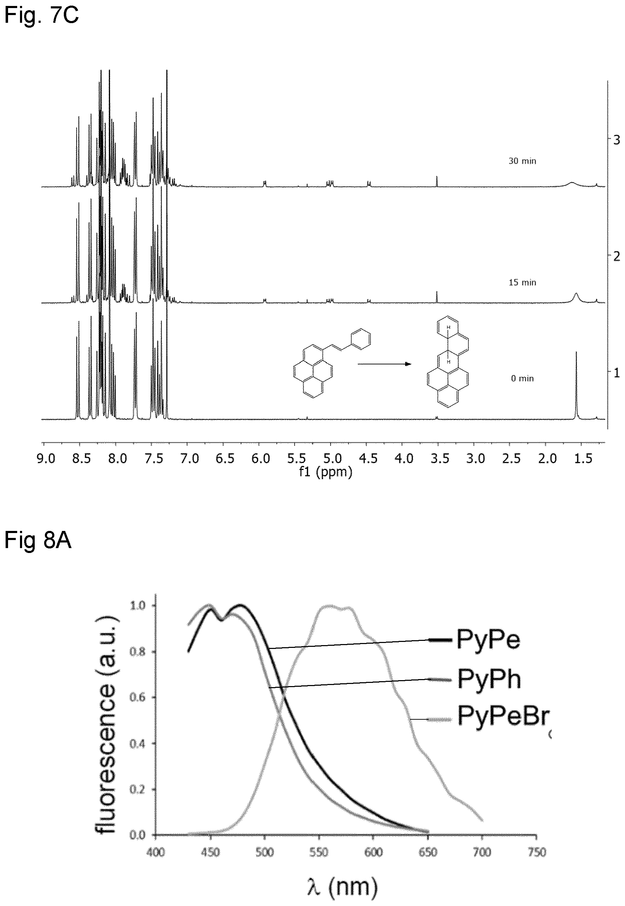

¹H-NMR spectra of 2mg of PyPh in deuterated chloroform (CDCl₃) after irradiation for 0, 15 and 30 min with excitation light at 400 nm (Xenon-arc lamp 300 Watt, FWHM 25 nm) are shown in Figure 7C. The observed formation of a new compound that might correspond to self-photobleached product that is shown in inset of this Figure 7C. The formation of this product is evidenced by the chemical shifts observed between 6.0ppm and 3.5ppm.

The self-bleaching of PyPh gives rise to products of similar nature as the dihydrophenanthrene adduct which was previously reported as a cis-stilbene self-bleaching product.

The new compounds PyPeBr as well as the PyPe and PyPh fluorophores were tested for their suitability to dye living cells.

To this end, cytotoxicity tests using the MTT assay, a standard colorimetric assay for assessing cell metabolic activity known to the person skilled in the art, on melanoma OVA-B16 cells line were performed. PyPeBr lead to minor or non-visible signs of deterioration in cell activity during the swelling or uptake of the PyPeBr provided in solution at 50 µM concentration dissolved in a cell buffered water or water/buffer/DMSO mixtures. Cell growth of OVA-16 cells was unaffected after 1h and 24h of exposure of PyPeBr at this concentration.

When applied to living cells the dyes PyPe, PyPh and PyPeBr were readily taken up by HEK/OVA-B16 cells, using standard staining protocols applying the dyes for up to 1 hour and at concentrations of 10µM.

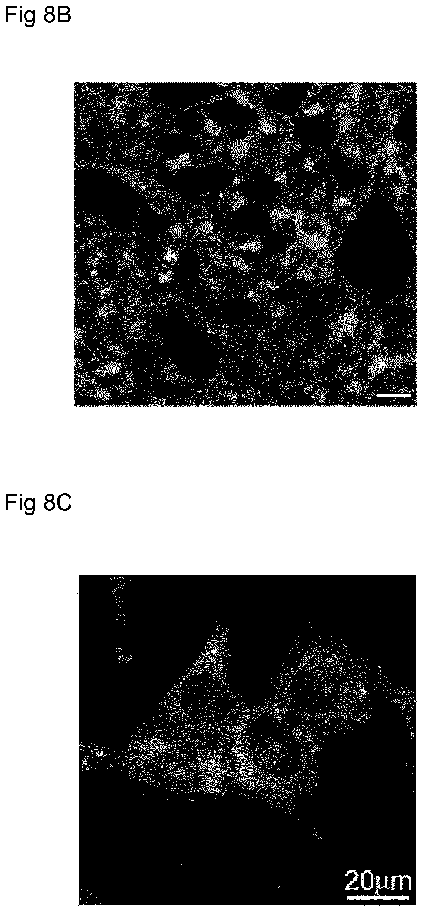

While scanning at 0.1 Hz over 30 minutes, using a Laser power of 2%, the signal intensity remained higher than 90% compared to t=0, indicating the long-term stability of the dye in living cells.
No morphological signs of cell toxicity were observed at these concentrations for PyPe and PyPh, while for PyPeBr, cytosol structure changes were observed at 10µM, and cells became round and started to detach at 50µM, indicating signs of moderate toxicity. The dyes were shown to stain mainly the cytosol. Figure 8C shows a PyPe staining of OVA-B16 cells. Similar staining patterns were observed for PyPeBr, which also stained the cytoplasm of the tested cell lines.

### Synthesis of (E)-1-allyl-4-(2-(pyren-1-yl)vinyl)pyridinium bromide (PyPeBr)

To a dried round bottom Schlenk flask equipped with a stirring bar 150 mg of (E)-4-(2-(pyren-1-yl)vinyl)pyridine was added. A dried CH₃CN 25mL solution was introduced sequentially under Argon flow to achieve a complete dissolution. Subsequently, an excess of dried 99 allyl bromide%, in this study 1ml, was added under an Argon atmosphere over a period of 30 min using a cannula while moderately stirring the mixture.

The mixture was gradually heated to a suitable temperature in the dark under Argon gas for a period of 12 hours. A suitable temperature range was 35 °C to 60°C.

After a successful reaction an orange-red precipitate was observed. The conversion rate of the reaction was checked by thin layer chromatography (TLC) using 50:50 Hexane-DCM as an eluent.

The collected precipitate was filtrated and washed 3 times with n-pentane. The washed precipitate was then dried under reduced pressure for 3 hours resulted in a titled compound with 98% yield. High-resolution mass spectrometry (HR-MS) was used to analyse the product, which was identified by a High-Resolution Mass Spectrometry Highest intensity monoisotopic weight at 346.15863. Additional peaks indicated the multi-isotopic weight related to Bromine anion.

### Synthesis of (E)-4-(2-(pyren-1-yl)vinyl)pyridine (PyPe)

1-Pyrenecarboxaldehyde (1 g, 4.35 mmol) and 4-picoline (0.65 g, 6.90 mmol) were placed in a dry round-bottom Schlenk flask which was flashed before a few times with Argon. Freshly distilled dry acetic anhydride was added (15 ml) with a cannula and the reaction mixture was heated under reflux for the subsequent 12h under vigorous stirring. Completeness of the reaction was tested by TLC, using THF/Hexane (1:5) as an eluent.

Upon completion of the reaction the reaction mixture was cooled to ambient temperature, and was left stirring for another 12h which led to the precipitation of the yellowish residue, which was collected by filtration, washed with large amount of the distilled water until neutral pH, and washed with n-hexane in a final washing step. The washed precipitate was dried under reduced pressure. The yield of the dried product was about 72%.

### Synthesis of [Zn(PyPe)Cl₂]·DCM (1)

Compound 2 was synthesized by mixing anhydrous ZnCl₂ (6.8 mg, 0.05 mmol) with PyPe (30.5 mg, 0.1 mmol) for 1 h orange precipitate formed, which was collected washed with n-pentane, dried and used for further analyses. Crystals of the compound were obtained by self-diffusion of component over one week. The product yield was about 64%.

### Optical properties of PyPe and PyPeBr

Emission maxima and absorption, lifetime and quantum yield of PyPe are presented in Table 1.

| Solvent | λ^{em} ₘₐₓ, nm^{a} | λ^{abs} ₘₐₓ, nm | Lifeti me,^{b} τ_{avr.}ns | Φ_{f}, ^{c} | *k*ᵣ [10⁸ s^{- 1}] | *K*ₙᵣ [10⁸ s⁻¹] |
|---|---|---|---|---|---|---|
| DCM | 623 | 412, 306 | 1.72 | 0.59 | 3.43 | 2.38 |
| THF | 613 | 413, 331,307 | 2.26 | 0.51 | 2.26 | 2.17 |
| c-Hex | 416, 438 | 403, 336, 302 | 7.58 | 0.64 | 0.84 | 0.47 |
| DMF | 475, 508, 614 | 441, 301 | 3.5 | 0.91 | 2.60 | 0.26 |
| Toluene | 603 | 413, 332, 308 | 2.25 | 0.88 | 3.91 | 0.53 |
| CH₃CN | 616 | 360, 308 | 2.07 | 0.92 | 4.44 | 0.38 |
| CHCl₃ | 607 | 416, 307 | 1.65 | 0.89 | 5.44 | 0.66 |
| MeOH | 611 | 374, 298 | 2.6 | 0.83 | 3.19 | 0.65 |

| | | | | | | |
|---|---|---|---|---|---|---|
| ^{a} λₑₓ = 405 nm, except for c-Hex 370 nm; ^{b} Multiexponential lifetime represented with τ_{avr.}; ^{c} determined using integrating sphere; | | | | | | |

Emission maxima and absorption, lifetime and quantum yield of PyPeBr are presented in Table 2.

| Solvent | λ^{em} max, nm | λ^{abs} max, nm | Lifetime, ^{a} τ_{avr.}ns | Φ_{f} ^{b} | *k*ᵣ [10⁸ s⁻¹] | *K*ₙᵣ [10⁸ s⁻¹] |
|---|---|---|---|---|---|---|
| DCM | 622 | 492 | 4.5 | 0.16 | 0.36 | 1.87 |
| THF* | 617 | 447 | 3.18 | 0.02 | 0.06 | 3.08 |
| c-Hex* | 416, 438 | 370 | 2.09 | 0.02 | 0.10 | 4.69 |
| DMF | 475, 615 | 393, 442 | 2.52 | 0.55 | 2.18 | 1.79 |
| Toluene* | 606 | 455 | 2.35 | 0.01 | 0.04 | 4.21 |
| CH₃CN | 621 | 398, 440 | 2.38 | 0.25 | 1.05 | 3.15 |
| CHCl₃ | 609 | 396, 444 | 1.58 | 0.99 | 6.27 | 0.06 |
| MeOH | 614 | 395, 444 | 1.95 | 0.74 | 3.79 | 1.33 |

| | | | | | | |
|---|---|---|---|---|---|---|
| *PyPeBr is poorly soluble in the indicated solvents, ^{a} Multiexponential lifetime represented with τ_{avr.}; ^{b} determined using integrating sphere; | | | | | | |

### Materials and measurements

4-picoline and 2-pyrene carbaldehyde, sodium hydroxide, tetrabutylammonium bromide, and allyl bromide were obtained from Sigma-Aldrich, ZnCl₂, diethyl benzylphosphonatewas purchased from TCI and used without further purification, acetic anhydride was freshly distilled, MeOH and DCM were used as received.

¹H-NMR was recorded on 400 MHz (Brucker DRX 400) and ¹³C-NMR spectra were recorded on Bruker 100 MHz spectrometer at room temperature with d₆-DMSO as a solvent and tetramethylsilane (TMS) as the internal standard. Mass spectra were performed on the Bruker-ION Trap Ms. Fluorescence spectra were measured on an Edinburgh
Instruments FS5 Spectrofluorometer. Absorption spectra were determined on a Perkin Elmer UV/VIS Lambda 2 (190-1100 nm) spectrophotometer. IR spectra were recorded on a Perking Elmer FT-IR spectrometer Frontier 4000-400 cm⁻¹. Single crystal structures were obtained from a STOE-IPDS II (Single crystal diffractometer λ-Mo), solved and refined using SHELXT software package.

## Claims

1. A fluorophore compound represented by any of the following General Formula P1, or General Formula P2 , or General Formula P3 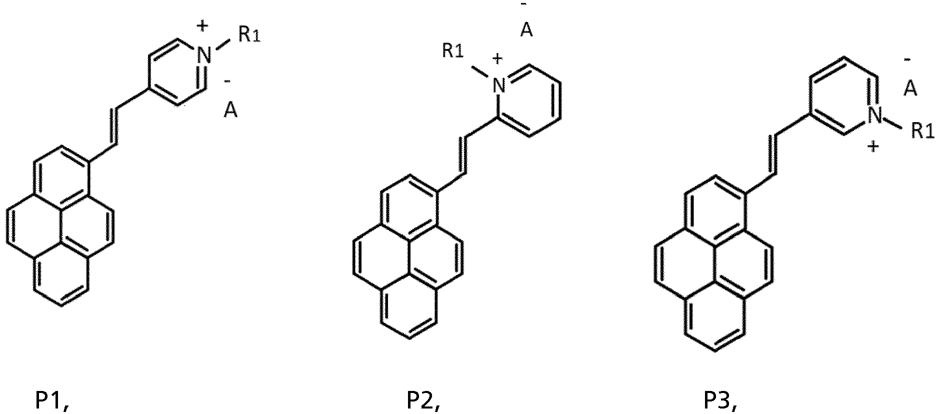
wherein R₁, denotes a methyl-group, or a linear or branched saturated or unsaturated alkyl group having from 2 to 10 carbon atoms, preferably having 2, 3, 4, 5, or 6 carbon atoms, optionally substituted by 1, 2 or 3 groups independently selected from OR₂, NR₂R_{2'}, SR₂, an aryl group, an heteroaryl group, a saturated or unsaturated cycloalkyl group having from 3 to 7 carbon atoms, or a CO₂R₃ group,
wherein R₂ and R_{2'} independently from each other denote H, a linear or branched saturated or unsaturated alkyl group having from 1 to 10 carbon atoms, preferably 1, 2, 3, 4, or 5 carbon atoms, optionally substituted by 1 to 3 groups independently selected from an aryl group, an heteroaryl group, or a saturated or unsaturated cycloalkyl group having from 3 to 7 carbon atoms, biotin, streptavidin, or avidin, and
wherein R₃ denotes H, a linear or branched saturated or unsaturated alkyl group having from 1 to 10 carbon atoms, preferably 1, 2, 3, 4, or 5 carbon atoms, optionally substituted by 1 to 3 groups independently selected from an aryl group, an heteroaryl group, or a saturated or unsaturated cycloalkyl group having from 3 to 7 carbon atoms, biotin, streptavidin, or avidin, and
wherein A⁻ denotes an anion selected from the group consisting of F, Cl⁻, Br⁻ , I⁻, tetrafluoroborate (BF₄⁻), hexafluorophosphate (PF₆⁻), triflate, tosylate, acetate, SO₄ ²⁻, OH⁻.

2. The compound of claim 1, wherein the compound is an allyl-pyridinium-4-yl anion, for example the para-isomer of the allyl-pyridinium-4-yl anion represented by Formula P1.1 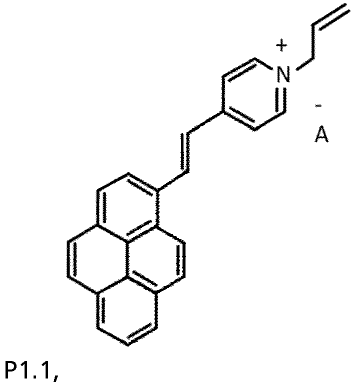 the ortho-isomer of the allyl-pyridinium-4-yl anion, or the meta isomer of the allyl-pyridinium-4-yl anion.

3. The compound of claim 1, wherein the compound is a (E)-1-(3-R4-oxy-3-oxopropyl)-4-(2-(pyrene-1-yl)vinyl)pyrdin-1-ium salt of General Formula P1.2, 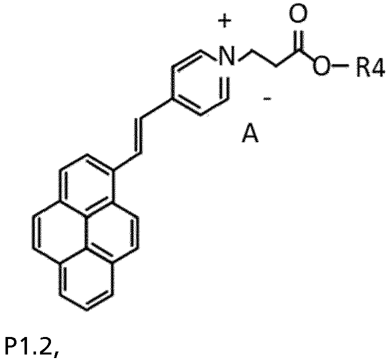
the ortho-isomer of the allyl-pyridinium-4-yl anion, or the meta isomer of the (Z)-1-(3-R4-oxy-3-oxopropyl)-4-(2-(pyrene-1-yl)vinyl)pyrdin-1-ium salt,
wherein A⁻ is as above defined and wherein R₄ denotes biotin, streptavidin or avidin.

4. The compound of any of claims 1 to 3, wherein the A⁻ is selected from a group consisting of bromide (Br⁻), chloride (Cl⁻), triflate, tosylate, acetate, sulphate (SO₄⁻), and nitrate (NO₃⁻).

5. The compound of any of claims 1 to 4, having a decrease in absorption of light at an excitation wavelength of less than 20%, or less than 15% or less than 10% after 5h or after 24h of irradiation with light at said excitation wavelength.

6. Composition comprising the compound represented by the General Formulas P1, P2, or P3, the *E*- stereoisomer, the *Z*- stereoisomer, or a mixture thereof, preferably a chemical equilibrium of a mixture of the *E-*stereoisomer and the *Z*-stereoisomer of the compound represented by the General Formula P1, General Formula P2, or General Formula P3, 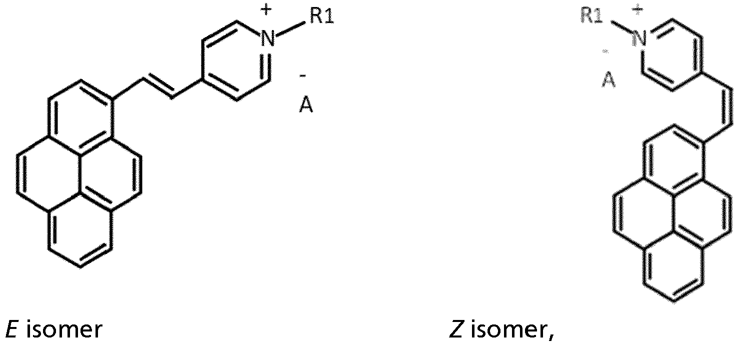 wherein R₁ is as above defined.

7. The composition of claim 6, wherein the solvent is water, methanol (MeOH), Acetonitrile (CH₃CN), chloroform (CHCl₃), dichloromethane (DCM), dimethylformamide (DMF), tetrahydrofuran (THF), toluene (Tol), c-hexane (c-HEX), or a mixture thereof.

8. A detection reagent comprising the compound of any of claims 1 to 5 conjugated to a molecular targeting agent, such as an antibody, for example a monoclonal antibody or a recombinant antibody, a polypeptide targeting agent, or a small molecular targeting agent.

9. Use of the compound of any of claims 1 to 5, or of the composition of any of claims 6 or 7, or of the detection reagent of claim 8 for imaging of cells, in particular living cells, cell components, biological tissues, and/or biomolecules.

10. An intermediate product for the synthesis of the compound of any of claims 1 to 6, said intermediate being represented by one of the following General Formulas P10.1, P10.2, or P10.3:

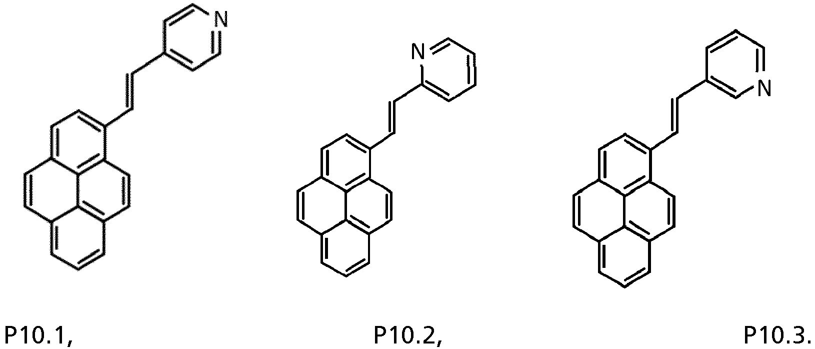

11. A method of producing the compound any of claims 1 to 6, comprising the steps of
(a) providing an intermediate product represented by the General Formula 10.1 to produce a compound of the General Formula P1, a product represented by the General Formula 10.2 to produce a compound of the General Formula P2, or a product represented by the General Formula 10.3 to produce a compound of the General Formula P3 dissolved in dried CH₃CN;
(b) mixing the dissolved intermediate product of the General Formula P10.1, P10.2, or P10.3 with preferably an excess amount of a product of the General Formula P11;
A - R1 P11,
wherein A denotes a substituent selected from a group consisting of F, Cl, Br, I, tetrafluoroborate (BF₄), hexafluorophosphate (PF₆), triflate, tosylate, acetate, SO₄, OH;
(c) providing dry acetic anhydride to the mixture;
(d) aging said mixture under dry conditions above ambient temperature for a period of time, preferably at a temperature ranging from 50°C to 80°C;
(e) cooling said aged mixture to ambient temperature to precipitate the product represented by the General Formula P1, represented by the General Formula P2, or represented by the General Formula P3;
wherein R₁, denotes a methyl-group, or a linear or branched saturated or unsaturated alkyl group having from 2 to 10 carbon atoms, preferably having 2, 3, 4, 5, or 6 carbon atoms, optionally substituted by 1, 2 or 3 groups independently selected from OR₂, NR₂R_{2'}, SR₂, an aryl group, an heteroaryl group, a saturated or unsaturated cycloalkyl group having from 3 to 7 carbon atoms, or a CO₂R₃ group,
wherein R₂ and R_{2'} independently from each other denote H, a linear or branched saturated or unsaturated alkyl group having from 1 to 10 carbon atoms, preferably 1, 2, 3, 4, or 5 carbon atoms, optionally substituted by 1 to 3 groups independently selected from an aryl group, an heteroaryl group, or a saturated or unsaturated cycloalkyl group having from 3 to 7 carbon atoms, biotin, streptavidin, or avidin, and
wherein R₃ denotes H, a linear or branched saturated or unsaturated alkyl group having from 1 to 10 carbon atoms, preferably 1, 2, 3, 4, or 5 carbon atoms, optionally substituted by 1 to 3 groups independently selected from an aryl group, an heteroaryl group, or a saturated or unsaturated cycloalkyl group having from 3 to 7 carbon atoms, biotin, streptavidin, or avidin, and wherein steps at least (b) to (d) are performed under the exclusion of oxygen.

12. The method of claim 11, wherein at least steps (b) to (d) are performed under the exclusion of light.

13. The method of claim 11 or 12, wherein the compound of the General Formula P11 is allyl-bromide of the General Formula P11.1.

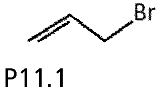

14. The method of claims 11 to 13, wherein the product represented by the General Formula P1, represented by the General Formula P2, or represented by the General Formula P3, is washed, for example with n-pentane, and dried, for example by applying reduced pressure, to obtain a dried final product represented by the General Formula P1, represented by the General Formula P2, or represented by the General Formula P3, respectively.

15. The method of claim 14, wherein the yield of the dried final product represented by the General Formula P1, represented by the General Formula P2, or represented by the General Formula P3 is at least 90%, or at least 95% or at least 98% of the amount of the provided intermediate product of General Formula P10.1, of General Formula 10.2, or of General Formula 10.3 respectively.

16. The method of any of claims 11 to 15, wherein the production of the intermediate product represented by the General Formula P10.1, by the General Formula 10.2, or by the General Formula 10.3 comprises the following steps:
(a.1) mixing the products of General Formula P20 and of General Formula P21.1, P21.2, or P21.3 under dry reaction conditions 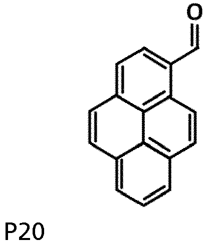 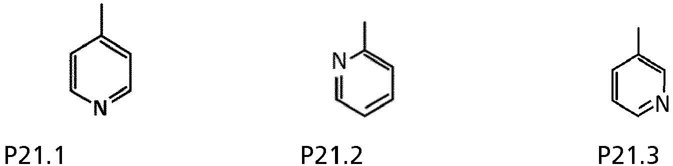
(a.2) providing dry acetic anhydride to the mixture;
(a.3) aging said mixture for a period of time at a temperature of 80°C to 120°C;
(a.4) cooling said aged mixture to ambient temperature to precipitate the intermediate product represented by the General Formula 10.1, respectively by the General Formula 10.2, or the General Formula 10.3.
